# EUROPEAN PATENT APPLICATION

(11) **EP 2 985 343 A1**
(43) Date of publication of application: **17.02.2016**
(21) Application number: 14002800.2
(22) Date of filing: 11.08.2014
(51) Int. Cl.: C12N 5/0789

(54) **In vitro-co-culturesystem**

(71) Applicant: Karlsruher Institut für Technologie, 76131 Karlsruhe (DE); Ruprecht-Karls-Universität Heidelberg, 69117 Heidelberg (DE)
(72) Inventor: Gottwald, Eric, 76149 Karlsruhe (DE); Giselbrecht, Stefan, 76187 Karlsruhe (DE); Wuchter, Patrick, 69118 Heidelberg (DE); Saffrich, Rainer, 69198 Schriesheim (DE)
(74) Representative: Müller-Boré & Partner Patentanwälte PartG mbB

(57) **Abstract**

The present invention relates to a three-dimensional (3D) model of a hematopoietic stem and progenitor cell (HSPC) niche, that comprises the coculture of human HSPC and human mesenchymal stromal cells in a defined 3D environment and thereby procures vital stem cell functions.

## Description

The present invention relates to a three-dimensional (3D) model of a hematopoietic stem and progenitor cell (HSPC) niche, that comprises the co-coculture of human HSPC and human mesenchymal stromal cells in a defined 3D environment and thereby procures vital stem cell functions.

Many attempts have been made to develop in vitro systems that can control hematopoietic stem cell proliferation and maintenance. For example, Dexter already in 1976 introduced a liquid culture system of small glass bottles containing femoral bone marrow cells of mice. The cells were cultured over a period of 3 weeks in which a layer of adherent cells, comprising fibroblastoid, epitheloid, and phagocytic mononuclear cells, was formed. After a "recharging" with fresh syngeneic femoral bone cells the production of hematopoietic stem cells for several months could be observed showing the importance of co-culture conditions for the preservation of hematopoietic competence. Although several successful attempts have been made to isolate and establish stromal cell lines for the study of some aspects of the regulation of hematopoietic stem cell proliferation and differentiation, the use of primary cells circumvents disadvantages that may be related to the use of cell lines.

Mesenchymal stromal cells (MSC) represent one of the major cellular determinants of the bone marrow niche. Numerous studies clearly demonstrated that cellular interaction with the niche cells plays a significant role for mobilization, homing and maintenance of stemness of hematopoietic stem and progenitor cells (HSPC). One of the major determinants for maintaining stem cell properties seems to be direct cell-cell-contact with the surrounding niche cells.

MSC have been used as a feeder layer in various 2D-co-culture systems with human HSPC to examine the regulation of these stem cell functions. However, 2D-systems might not adequately reflect the *in vivo*-situation.

Thus, the problem underlying the present invention is to provide a suitable microenvironment for HSPC that resembles the physiologic *in vivo*-situation and allows the long-term culture of these cells while maintaining their characteristic stem cell functions ("stemness").

The solution to the above technical problem is achieved by the embodiments characterized in the claims.

In particular, the present invention relates to a three-dimensional (3D) model of a hematopoietic stem and progenitor cell (HSPC) niche comprising:
(a) a microcavity array having at least one microcavity
(b) a 3D mesh formed by HSPC-supporting stroma cells in the at least one microcavity,

Within the scope of the invention, the term "cell" is a generic term and encompasses individual cells, tissues, primary cells, stem cells, continuous cell lines, induced pluripotent stem cells and/or genetically engineered cells. In a preferred embodiment of the present invention, cells are mammalian cells including those of human origin, which may be primary cells derived directly from a donor, primary cells derived from a tissue sample, diploid cell strains, transformed cells or established cell lines. Mammalian cells can include human and non-human cells alike. In a preferred embodiment of the present invention, the cells are human cells.

In a preferred embodiment of the present invention the HSPC-supporting stroma cells are mesenchymal stromal cells (MSC) or osteoblasts. In a preferred embodiment of the present invention, the HSPC-supporting stroma cells are isolated from human bone marrow. As an alternative, stromal cell lines, e.g. HUVEC, may be used. Alternatively, any kind of cells can be used for the co-culture with the HSPCs.

According to the present invention, the HSPC-supporting stroma cells and the HSPCs may be isolated and/or cultivated before being brought into contact with the microcavity array. Methods for isolation and cultivation of HSPC-supporting stroma cells and HSPCs are known in the prior art. In a preferred embodiment of the present invention, the HSPC-supporting stroma cells are isolated from blood and/or bone marrow, more preferably from the mononuclear cell fraction from blood and/or bone marrow. The isolation may involve density centrifugation, selection via plastic adherence and selection using surface markers, like for example CD73+, CD90+, CD105+ and/or a combination of markers. In a preferred embodiment of the present invention, the HSPC are isolated from mobilized peripheral blood (mPB; peripheral blood from donors who received G-SCF for mobilization of HSPC) and/or umbilical cord blood (CB), more preferably from the mononuclear cell fraction from mPB or CB. The isolation may involve density centrifugation and selection using surface markers, like for example CD34+, CD133+, and/or a combination of CD34+/CD38-.

In a preferred embodiment of the present invention, the 3D mesh formed by HSPC-supporting stroma cells in the at least one microcavity comprises about 2 to several thousand cells, more preferably about 100 to 10000 cells, most preferably about 500 to 5000 cells. In another preferred embodiment of the present invention, the 3D mesh formed by HSPC-supporting stroma cells in the at least one microcavity comprises at least 2, at least 50, at least 100, at least 200, at least 500, at least 1000, at least 2000, or at least 5000 cells.

The term "microcavity array" does not have any specific limitations, and relates, for example, to glass or an insoluble polymer material, which can be an organic polymer, such as polyamide or a vinyl polymer (e.g. poly(methyl)methacrylate, polycarbonate, polystyrene and polyvinyl alcohol, cyclo-olefin-polymer, or derivatives thereof), a natural polymer such as cellulose, dextrane, agarose, chitin and polyamino acids, or an inorganic polymer, such as glass or metallohydroxide. In another embodiment the term "microcavity array" relates to non-biodegradable polymers, preferably polymethacrylate, polycarbonate, polystyrene, polyvinylalcohol, cyclo-olefin-polymer, cyclo-olefin-co-polymer, and / or derivatives thereof. In a further embodiment the term "microcavity array" relates to biodegradable polymers, preferably polylactide, polyglycolide, polycaprolactone, and / or derivatives.

In a preferred embodiment of the present invention, the microcavity array comprises polymethylmethacrylate and polycarbonate. In another preferred embodiment of the present invention, the microcavity array is completely made of or equipped with a polycarbonate membrane bonded to the back of the array which forms the at least one microcavity or manufactured by a porous membrane itself.

The microcavity array can be in incorporated into a chip, inserts, strips, film, or plates, such as microtiter plates or microarrays.

In a preferred embodiment of the present invention, the microcavity array has about 1 to 100000, more preferably about 10 to 10000, more preferably about 100 to 1000, and most preferably about 300 to 800 microcavities formed on its surface. In a particularly preferred embodiment of the present invention, the microcavity array has about 625 microcavities formed on its surface. In another preferred embodiment of the present invention, the microcavity array has at least 1, at least 2, at least 4, at least 6, at least 8, at least 10, at least 12, at least 24, at least 48, at least 50, at least 96, at least 100, at least 200, at least 384, at least 500, at least 1000, at least 1536, at least 2000, or at least 5000 microcavities formed on its surface.

In a preferred embodiment of the present invention, the microcavity array comprises a moulded body as disclosed in European patent EP 1 768 833 B1. In another preferred embodiment of the present invention, the microcavity array comprises or consists of an input for a microtiter plate as disclosed in German utility model DE 20 2006 017 853 U1. In another preferred embodiment of the present invention, the microcavity array comprises or consists of a bioreactor as disclosed in German utility model DE 20 2006 012 978 U1. In another preferred embodiment of the present invention, the microcavity array comprises or consists of the p-3^{D}-KITChip, the f-3^{D}-KITChip, or the r-3^{D}-KITChip as disclosed in E. Gottwald et al. / Z. Med. Phys. 23 (2013) 102-110.

In another preferred embodiment of the present invention, the microcavity array has about 1 to 10000, more preferably about 5 to 1000, most preferably about 10 to 600 microcavities per cm² formed on its surface. In another preferred embodiment of the present invention, the microcavity array has at least 1, least 2, least 5, least 10, at least 50, at least 100, at least 200, at least 500, at least 1000, at least 2000, or at least 5000 microcavities per cm² formed on its surface.

The term "microcavity" as used herein means a three-dimensional surface structure on one or two surfaces of the microcavity array according to the present invention. The microcavities may have any suitable form and size. The microcavity is open to at least one side. The micracavity shape, depth and diameter are highly variable in range, for example the microcavity may be round. In one preferred embodiment at least one side of the microcavity is porous, in a more preferred embodiment the microcavity as used herein has a porous bottom. The microcavity may be a structure with an open side and a round or rectangular bottom opposite to the open side. In a preferred embodiment of the present invention, the microcavity has a rectangular bottom and four rectangular walls. In a particularly preferred embodiment of the present invention, the microcavity has a rectangular base area and four rectangular walls, wherein the bottom and the four rectangular walls are of the same size.

In a preferred embodiment of the present invention, the thickness of the microcavity walls is 0.5 to 500 µm, more preferably of 10 to 100 µm, and most preferably of 30 to 70 µm.

In a preferred embodiment of the present invention, the microcavity has a volume of 1 to 100 nl, more preferably of 10 to 50 nl, and most preferably of 20 to 40 nl. In a particularly preferred embodiment of the present invention, the microcavity has a volume of 27 nl.

In another preferred embodiment of the present invention, the microcavity has a size of 20 to 1000 µm x 20 to 1000 µm x 20 to 1000 µm (height x depth x width), more preferably 100 to 600 µm x 100 to 600 µm x 100 to 600 µm (height x depth x width), most preferably 200 to 400 µm x 200 to 400 µm x 200 to 400 µm (height x depth x width). In a particularly preferred embodiment of the present invention, the microcavity has a size of 300 µm x 300 µm x 300 µm (height x depth x width).

In a preferred embodiment of the present invention, the bottom of the chip is porous. In a more preferred embodiment of the present invention, the bottom of the chip has about 1 to 10 million, more preferably about 1000 to 5 million, most preferably about 1 million to 2 million pores per cm².

In another preferred embodiment of the present invention, the bottom of the at least one microcavity is porous. In a preferred embodiment of the present invention, the bottom of the at least one microcavity has about 10⁴ to 10⁷ pores per cm², more preferably 10⁵ to 5 x 10⁶ pores per cm², and most preferably 5 x 10⁵ to 2 x 10⁶ pores per cm². In a particularly preferred embodiment of the present invention, the bottom of the at least one microcavity has about 10⁶ to 2 x 10⁶ pores per cm².

In another preferred embodiment of the present invention, the bottom of the at least one microcavity has about 1 to 1 x 10⁵, more preferably about 10 to 10⁴, most preferably about 100 to 2000 pores per mm².

In another preferred embodiment of the present invention, the pores in the bottom of the at least one microcavity have an average diameter of about 0.01 to 10 µm, more preferably about 0.5 to 5 µm, most preferably about 1 to 3 µm. In a particularly preferred embodiment of the present invention, the pores in the bottom of the at least one microcavity have an average diameter of about 3 µm.

In a preferred embodiment of the present invention, the bottom of the at least one microcavity is a membrane, more preferably a microporous membrane. The membrane may comprise polycarbonate, more preferably the membrane consists of polycarbonate.

The present invention further relates to the 3D model of a HSPC niche as described herein further comprising HSPC. In a preferred embodiment of the present invention, the HSPC are attached to the 3D mesh formed by HSPC-supporting stroma cells in the at least one microcavity via cell-to-cell interaction. In a preferred embodiment, the HSPC and the HSPC-supporting stroma cells are derived from the same species. In a more preferred embodiment, the HSPC and/or the HSPC-supporting stroma cells are human.

In a preferred embodiment, the HSPC are human. In another preferred embodiment, the HSPC primary cells are derived directly from a donor or primary cells derived from. a tissue sample. In a particularly preferred embodiment of the present invention, the HSPC are human HSPC isolated from umbilical cord blood, mobilized peripheral blood or from bone marrow.

In a preferred embodiment of the present invention, the HSPC have a purity of at least 80%, preferably at least 90%, more preferably at least 95% HSPC CD34⁺ cells.

The present invention provides a method of cultivating hematopoietic stem and progenitor cells (HSPC) comprising the step of cultivating HSPC in the three-dimensional (3D) model of a human HSPC niche as defined herein.

In a preferred embodiment, the method of cultivating HSPC is a method for preserving stem cell properties of cultivated HSPC. The present invention provides a method of cultivating HSPC is a method for preserving stem cell properties of cultivated HSPC comprising the step of cultivating HSPC in the 3D model of a HSPC niche as defined herein.

In a preferred embodiment, the method as defined herein comprises the steps of:
(a) providing a microcavity array having at least one microcavity,
(b) seeding a mixture of HSPC-supporting stroma cells and HSPC at the same time into the at least one microcavity,
(c) cultivating the cells of step (b).

In another preferred embodiment, the method as defined herein comprises the steps of:
(a) providing a microcavity array having at least one microcavity having a porous bottom,
(b) seeding HSPC-supporting stroma cells in the at least one microcavity,
(c) cultivating the HSPC-supporting stroma cells of step (b) until a 3D mesh is formed,
(d) seeding HSPC into the 3D mesh formed by the HPC-supporting stroma cells in step (c), and
(e) cultivating the HSPC of step (d).

As an alternative, HSPC and HSPC-supporting stroma cells can be seeded at the same time together and a 3D mesh of stroma cells with the HSPC integrated herein is formed.

In a preferred embodiment of the method as defined herein, the HSPC-supporting stroma cells are mesenchymal stromal cells (MSC).

In a preferred embodiment, the method according to the present invention further comprises the step of purifying HSPC CD34+ cells by positive selection prior to step (b). In a particularly preferred embodiment, the step of purifying HSPC CD34+ cells by positive selection e.g. via magnetic-activated cell sorting (MACS) or by means of fluorescent activated cell sortin (FACS) is carried out until the HSPC have a purity of at least 80%, preferably at least 90%, more preferably at least 95% HSPC CD34+ cells.

In a preferred embodiment of the method as defined herein, the cultivation of cells is carried out in a closed circulation loop system comprising the microcavity array in a bioreactor, a cassette pump, a gas mixing station, and a medium reservoir.

In a preferred embodiment of the present invention, the microcavity array comprises or consists of the p-3^{D}-KITChip, the f-3^{D}-KITChip, or the r-3^{D}-KITChip as disclosed in E. Gottwald et al. / Z. Med. Phys. 23 (2013) 102-110 and the bioreactor is the bioreactor setup disclosed in Gottwald et al., LAB CHIP 2007; 7(6): 777-785. The microcavity array may be coated with an agent which facilitates the adhesion and/or growth of cells.

In a preferred embodiment of the method according to the present invention, the cells are cultivated in a microbioreactor system that is actively perfused and/or superfused with medium.

In a preferred embodiment of the method as defined herein, the cultivation of cells carried out using superfusion of the cells with medium, and/or using perfusion of the cells with medium. In a particularly preferred embodiment of the method as defined herein, during superfusion the medium flows in parallel to the array surface. In a particularly preferred embodiment of the method as defined herein the chip has a porous bottom and during perfusion the medium enters the chip through the porous bottom of the at least one microcavity. In a more preferred embodiment the superfusion of the cells with medium is carried out with a flow regime at about 62.5 to 800 µl/min flow, more preferably at about 200 to 600 µl/min flow, more preferably at about 300 to 500 µl/min flow, most preferably at about 400 µl/min flow. In another more preferred embodiment the perfusion of the cells with medium is carried out with a flow regime at about 62.5 to 800 µl/min flow, more preferably at about 200 to 600 µl/min flow, more preferably at about 300 to 500 µl/min flow, most preferably at about 400 µl/min flow. The flow rate of the superfusion may be the same or different.

There is no particular limitation to the media, reagents and conditions used for culturing the cells in a cell culture as described herein. In a preferred embodiment, the cultivation of HSPC is carried out using LTBMC medium which is a basal HSPC culture medium consisting of 75% Iscove's modified Dulbecco's medium, 12.5% FCS, 12.5% horse serum, supplemented with 2 mM L glutamine, 100 U/ml penicillin/streptomycin and 0.05% hydrocortisone 100.

In a preferred embodiment of the method as defined herein, at least one supplement of the culture medium is added for a limited time and/or in a limited amount. In a more preferred embodiment, at least one supplement is selected from the group consisting of cytokines, chemokines, inhibitors and combinations thereof.

In a preferred embodiment, the cultivation of HSPC-supporting stroma cells and of HSPC is carried out between 15°C to 45°C, preferably between 20°C and 40°C, more preferably between 35°C and 40°C, and most preferably at 37°C. In another preferred embodiment the cells are incubated at 37°C at an atmosphere containing 5% CO₂ and oxygen concentrations ranging from 0 to 100%, more preferably from 1 to 30%, and most preferably between 1 and 21%. It is within the knowledge of the person skilled in the art to choose the optimal parameters, such as buffer system, temperature and pH for the respective detection system to be used.

In a preferred embodiment of the method as defined herein, the HSPC-supporting stroma cells and/or the HSPC cells are harvested and/or analysed after the method according to the present invention.

In a preferred embodiment of the method as defined herein, the MSC and the HSPC are derived from the same species. In a more preferred embodiment of the method as defined herein, the MSC and the HSPC are of human origin.

The present invention further relates to a use of a three-dimensional (3D) model of a hematopoietic and progenitor stem cell (HSPC) niche as defined herein for cultivating HSPC.

The present invention further relates to a use of a three-dimensional (3D) model of a hematopoietic stem and progenitor cell (HSPC) niche as defined herein for preserving stem cell properties of cultivated HSPC.

The present invention further relates to a use of a three-dimensional (3D) model of a hematopoietic stem and progenitor cell (HSPC) niche as defined herein for monitoring the differentiation, the preferably stepwise differentiation, of HSPC. Methods for monitoring the differentiation of HSPC are well known in the art and include the use of differentiation media which may be followed by a colony-forming assay.

The present invention further relates to a use of a three-dimensional (3D) model of a hematopoietic stem and progenitor cell (HSPC) niche as defined herein as a microenvironment for cultivating HSPC. In a preferred embodiment of this use stem cell properties of cultivated HSPC are preserved and/or the differentiation of HSPC is monitored.

The present invention relates to a kit containing a three-dimensional (3D) model of a hematopoietic stem and progenitor cell (HSPC) niche as defined herein. The kit may further contain e.g. stroma cells, culture medium a buffer solution, reaction containers, and/or transfection reagents, when appropriate.

The present invention contains properties which are considered to be of importance for a meaningful *in vitro-*system: a defined geometry/niche size, a defined and controllable cell number, an organotypic 3D-mesh of human HSPC-supporting stromal cells, defined flow conditions, and a controllable gas partial pressure in the system. With the present invention a novel 3D-co-culture system has been developed which is a more realistic *in vitro*-model of the hematopoietic stem cell niche and relies on primary cells. This more organotypic *in vitro-*model can serve various purposes, such as to better understand fundamental questions of the regulation of the native niche, identify stepwise the process of stem cell differentiation, explore means to manipulate the niche, and to develop new therapeutic modalities.

The figures show:
Figure 1: 3^{D}-KITChip with porous membrane at the bottom.
Figure 2: ΔΔCt analysis of a HSPC/MSC-coculture under superfusion conditions (400 µl/min) in a 3^{D}-KITChip-containing bioreactor. * = p<0.05 Mann-Whitney U-test.

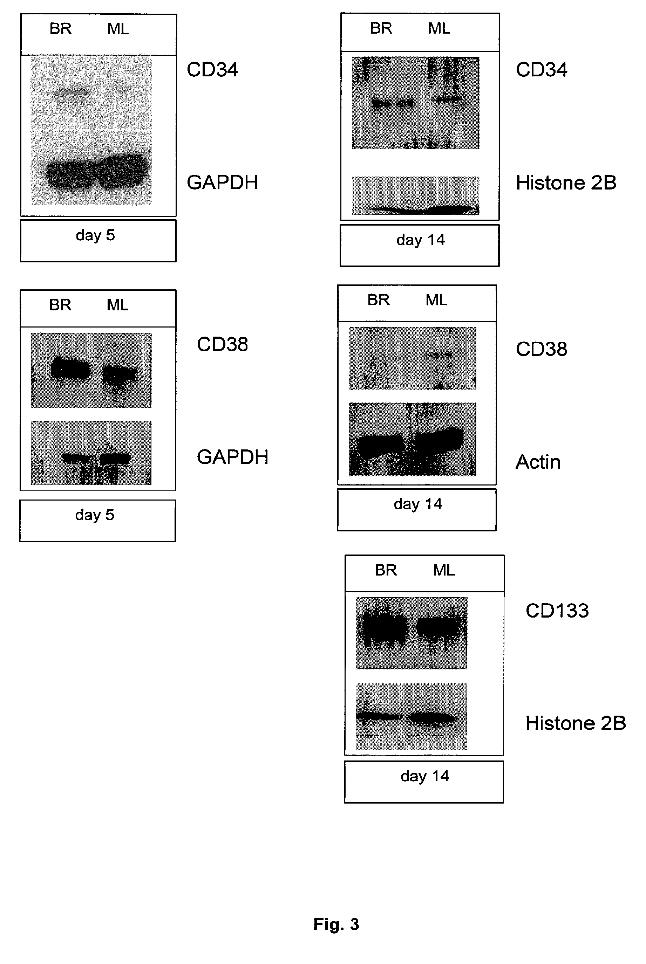

Figure 3: Western Blot analysis of CD34-, CD38-, CD133-, and GAPDH- or Histone 2B-expression. Monolayer and bioreactor samples were lysed after 5 and 14 days of the respective culture technique and subjected to western blot analysis.

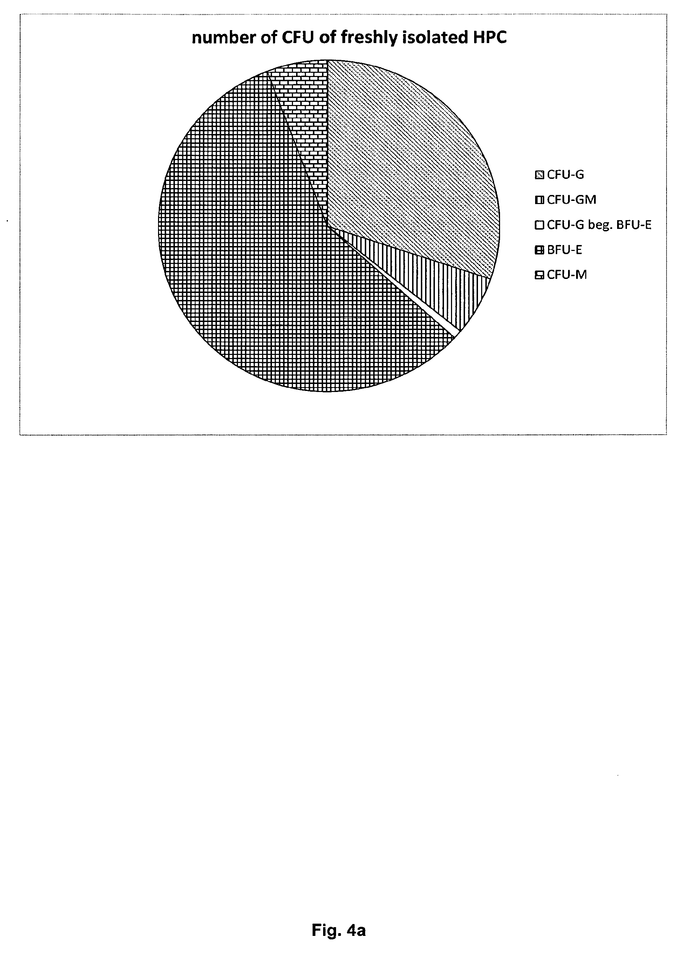

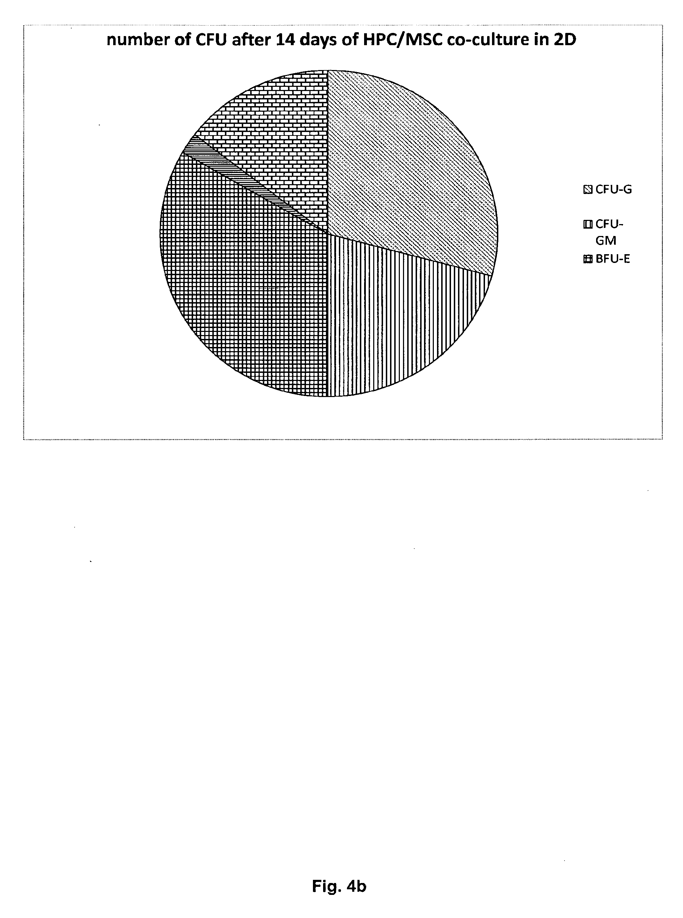

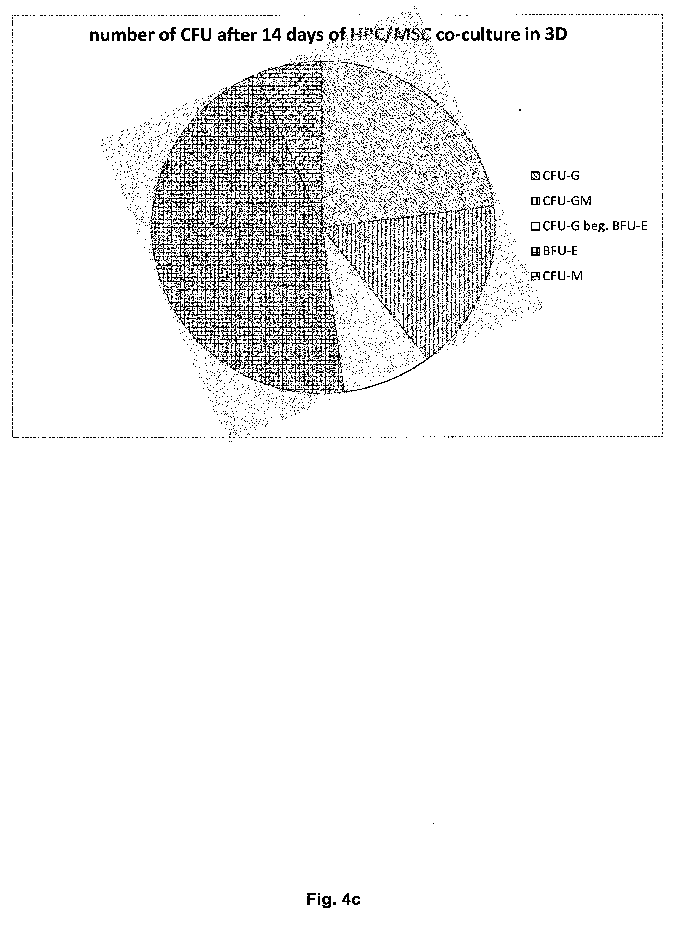

Figure 4: Comparison of colonies formed in the colony-forming assay. Freshly isolated HSCs and HSCs after 2D- or 3D-co-culture were analysed.

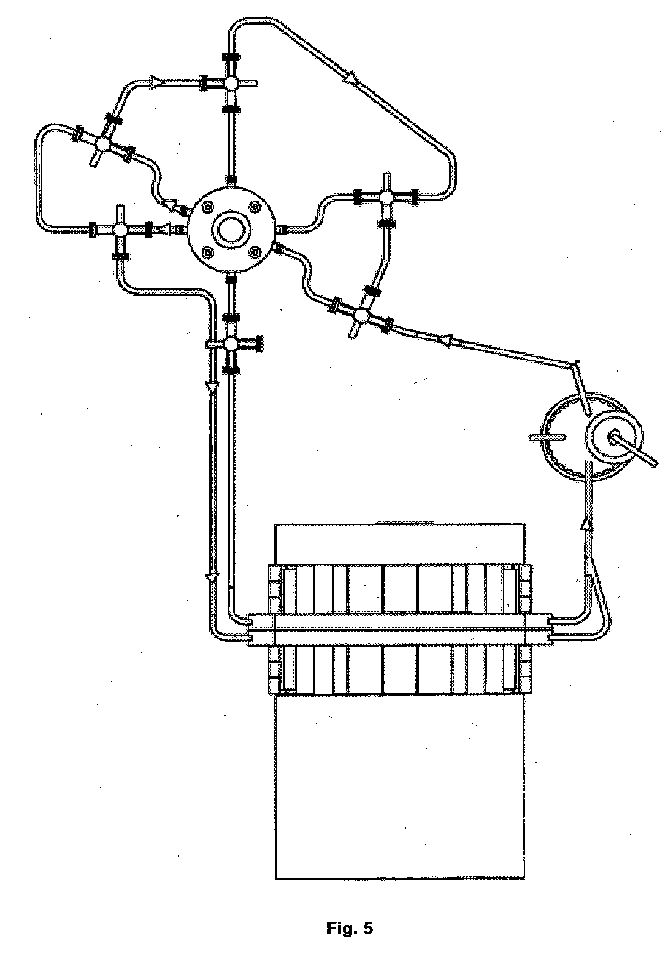

Figure 5: Bioreactor setup with bioreactor, medium reservoir and cassette pump.

The present invention will now be further illustrated in the following examples without being limited thereto.

### Example

Here, we present for the first time the development of this novel 3D-model system of the human hematopoietic stem cell niche and report about its impact upon HSC properties over a culture period of 14 days. In the first experimental series human HSPC and MSC could be cultured within the KITChip in a 3D-mesh while the whole chip was mounted in a microfluidic bioreactor. Within the closed circulation system, oxygen concentration, medium composition, and medium flow could be controlled precisely. The latter has been shown as being very uniform for all flow rates tested so far. We could show that the bioreactor system led to a better preservation of CD34 in human HSPC. Moreover, in colony-forming-unit-assays we could show that HSPC isolated from the bioreactor displayed nearly the same plasticity like freshly isolated CD34⁺-cells from cord blood compared to a monolayer reference culture.

### Results

### Bioreactor setup

The bioreactor for the housing of the 3^{D}-KITChip can be operated in various flow regimes. The two principally different ones are the superfusion and perfusion mode. Although already many attempts have been made to culture stem cells in microbioreactors, no attempts have been made with bioreactors and targeted capability of avoiding gradients or generating gradients of soluble factors and oxygen in defined way. By applying the superfusion flow regime in our bioreactor system, where the fluid flow is parallel to surface of the tissue, the supply with nutrients and gases in the depth of microcavities is realized by diffusion, thereby generating gradients. In contrast to this, in the perfusion mode the medium is flowing perpendicular to the surface of the tissue, meaning that it is flowing through the porous bottom of the chip through the tissue in each microcavity and thus avoiding gradients. First experiments with the HSPC/MSC co-culture showed no differences in the behavior of cells under perfusion conditions with 400 µl/min flow (data not shown) whereas superfusion with the same flow rate did. All experimental data presented in the work were thus generated with the superfusion flow regime at 400 µl/min flow.

### Real-Time RT-PCR

We analyzed the expression pattern of some hematopoietic and mesenchymal stromal markers as well some markers indicative of differentiation of the HSPCs into the lymphoid and myeloid lineage after a cultivation of 1, 5 and 14 days, respectively. As can be seen in the figures, obviously the HSPC population is divided into at least 2 subpopulations one of which expresses CD34 throughout the whole bioreactor culture indicating stem cell maintenance whereas the other subpopulation increases CD38-expression, indicating loss of stem cell characteristics.

This is line with findings of IL-7, CD14 and CD15-expression that indicates lymphoid and myeloid differentiation. However, the expression of stromal markers, such as CD90, CD105 and stem cell markers, such as c-kit and Nestin, showed a tendency to be increased during the culture period. Interestingly, cell-cell adhesion molecules presumably involved in stem cell maintenance, such as CXCR-4, showed a tendency to increase in expression up to day 14 which may be indicative of the necessity of three-dimensionality to express this marker. However, the expression of the ligand of CXCR-4, SDF-1, decreased in expression over the culture period and currently does not fit into the model that three-dimensionality enhances cell-cell adhesion molecule expression. On the other hand, it could be speculated that the reduction of SDF-1 reflects a down-regulation due to the sufficient availability of its cellular counterpart, CXCR4. Interestingly, the Cyclin Dependent Kinase inhibitor p21 in tendency is expressed slightly higher in the three-dimensional environment, pointing towards a decreased proliferation in the bioreactor environment.

### Western Blot

The gene expression suggested that CD34 and CD38 were both upregulated in superfused culture. We, therefore, performed western blots to elucidate the expression level on a protein basis. As can be seen in the figures, CD34-expression was higher at the days analyzed (5 and 14) in the three-dimensional bioreactor culture compared to the monolayer confirming the PCR-results and the notion that the HSPC-population is divided at least into two fractions, one of which keeps the CD34-expression high whereas the other is slightly losing the stem cell characteristics.

Depending on the source of stem cells, CD34 may not be expressed on all progenitor cells. An alternative stem cell marker is prominin-1 (CD133), which is expressed preferably on a subpopulation of CD34⁽⁺⁾ progenitor cells derived from various sources including fetal liver and bone marrow, adult bone marrow, cord blood, and mobilized peripheral blood.

Therefore, in addition we analysed the expression of CD133 to get an impression whether CD133 is differently expressed in the two types of cultures. The figures show the results, where it is apparent that, if at all, there is only a slightly higher expression of CD133 in the bioreactor culture. However, since it has been proposed that CD133-containing membrane microdomains might act as stem cell-specific signal transduction platforms and their reduction will somehow lead to cellular differentiation, the high expression of CD133 would explain the results obtained in the colony forming unit-assays where we could show a maintenance of stem cell plasticity after 14 days of bioreactor culture.

### Colony-Forming-Cell-Assay

We analyzed the plasticity of cord blood CD34+-cells by performing colony-forming-cell-assays. Freshly isolated HSPCs were compared with those recovered from the 2D- or 3D-culture. As can be seen, freshly isolated cells mainly formed erythrocytic burst forming unit (BFU-E, 57%), colony-foming units of granulocytes (CFU-G, 30%), and to a smaller extent macrophage colony forming units (CFU-M, 6%) macrophage, granulocyte-macrophage units (CFU-GM, 6%), granulocyte colony forming units with beginning burst forming units of erythrocytes and (CFU-G beg. BFU-E, 1 %).

After a period of 14 days either in 2D or 3D-bioreactor culture this distribution changed dramatically in monolayer culture in which the CFU-G colonies were determined to be 29%, the CFU-GM 21%, BFU-E 33%, the CFU-E 2%, and the CFU-M 15%. In contrast to the 2D-culture, the 3D-culture after 14 days showed no changes in the colony distribution. The CFU-G were determined to be 29%, the CFU-GM 17%, CFU-G beginning BFU-E 8%, the BFU-E 46% and the CFU-M 6%. These data indicate that the 3D-organotypic culture leads to the preservation of the plasticity degree observed in freshly isolated hematopoietic progenitor cells.

### Conclusions

It is known that co-culture of stromal cells from the bone marrow and human hematopoietic progenitor cells is able to maintain stemness to a higher degree and over a longer period of time as compared to suspension culture. We have established a novel 3D-model system of the hematopoietic stem cell niche using human primary hematopoietic progenitor cells and mesenchymal stromal cells in a perfused bioreactor environment. The chip-based bioreactor allows for a defined co-culture of cells in microcavities of 300 µm in depth, length and height. The closed circulation loop comprised the bioreactor, a peristaltic pump and a gas mixing station for optimal control of gas partial pressures. In addition the system allowed a defined medium flow with two different flow regimes: superfusion and perfusion with the major difference being the fact that in the first case the medium flows in parallel to the chip surface whereas in the other case the medium enters the chip from below resulting in a perpendicular flow with respect to the surface of the chip. The effect of the bioreactor setup was compared to a conventional 2D co-culture setup, consisting of a monolayer of mesenchymal stromal cells and hematopoietic progenitor cells on top.

We could demonstrate that in this chip-based 3D-culture system the HSPC and MSC could be kept viable for up to 21 days. Specific stem cell properties could be preserved for at least 14 days in vitro to a much higher degree as compared to standard co-culture conditions. Taken together, we could show that this stem cell niche model provided a more physiologic environment for CD34⁺ hematopoietic progenitor cells leading to enhanced stem cell maintenance and plasticity. The versatility of the system could serve various applications in the area of fundamental research as well as preclinical drug screening and toxicity testing (e.g. regarding tests for the European Union regulation REACH). Examples for useful applications in fundamental research could be the identification of soluble factors and cellular determinants responsible for maintaining stemness. In the clinical context, it could serve as a platform for the validation and preclinical testing of novel stem cell mobilization reagents or other reagents that affect hematopoiesis, e.g. erythropoiesis stimulating agents (ESAs).

### Experimental Setup

### Chip manufacturing and design

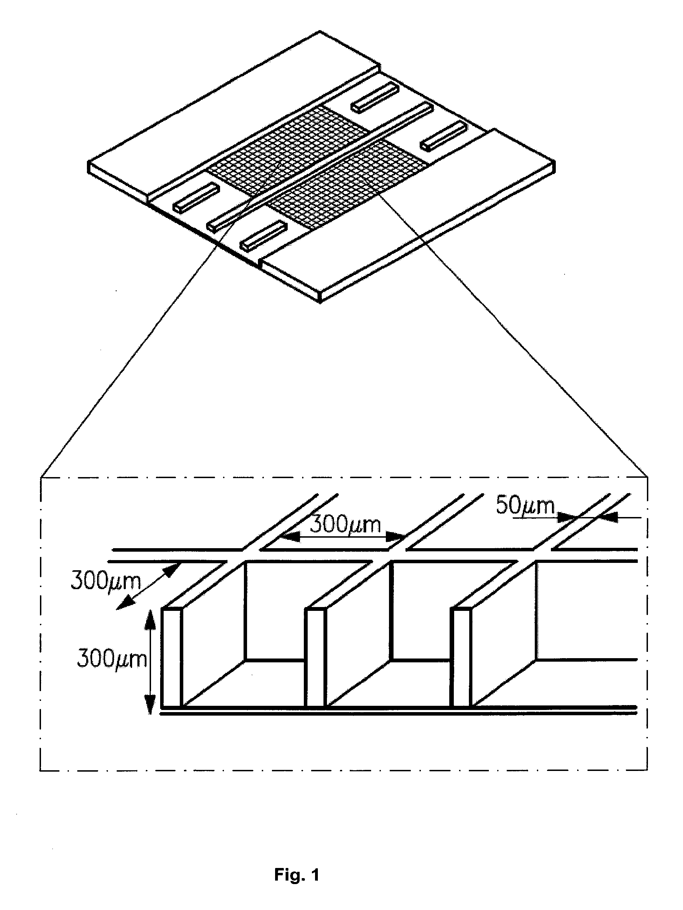

The 3^{D}-KITChip used for the experiments is the so-called f-3^{D}-KITChip (Fig. 1), one of the currently manufactured variants of the 3°-KITChip-family. Briefly, the manufacturing technique for this chip type combines a micro injection moulding/micro hot embossing and a solvent welding step of a microperforated membrane to the back of the chip. The chip produced by this process is characterized by an increased flexibility with regard to adjusting material properties and porosity of the bottom of the chip for customized cell culture applications. Prior to bonding, the residual layer of the molded chip was not only thinned down but instead was completely ablated resulting in a back-side opened chip. The bonding process is a solvent welding process intended to yield joints without any additional or foreign substances (e.g. adhesives) which is advantageous for achieving a biocompatible product.

### Bioreactor setup

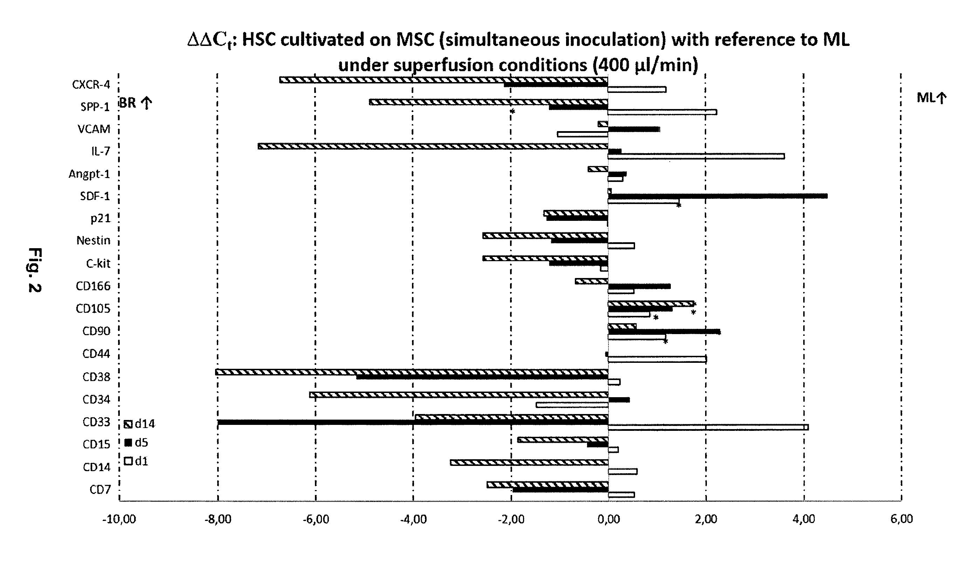

The bioreactor setup consisted of the 3^{D}-KITChip that was inserted into a bioreactor housing (Fig. 2), a medium reservoir and cassette pump.

### Isolation of human hematopoietic stem and progenitor cells

Hematopoietic stem and progenitor cells (HSPC) were isolated from umbilical cord blood after obtaining informed consent according to the guidelines approved by the Ethics Committee of the Medical Faculty of Heidelberg University. HSPC were isolated as described before (Wein, F., et al., N-cadherin is expressed on human hematopoietic progenitor cells and mediates interaction with human mesenchymal stromal cells. Stem Cell Res, 2010. 4(2): p. 129-39).

Briefly, mononuclear cells (MNC) were isolated by density gradient centrifugation on Ficoll-hypaque technique (Biochrom KG, Berlin, Germany). CD34+ cells were purified by positive selection with a monoclonal anti-CD34 antibody using magnetic microbeads on an affinity column with the AutoMACS system (all Miltenyi Biotec, Bergisch-Gladbach, Germany). Reanalysis of the isolated cells by flow cytometry revealed a purity of >95% CD34+ cells.

### Isolation of human mesenchymal stromal cells

Human mesenchymal stromal cells (MSC) were isolated from human bone marrow (BM) from healthy voluntary donors after obtaining informed consent according to the guidelines approved by the Ethics Committee of the Medical Faculty of Heidelberg University. MSC were isolated and expanded using standardized culture conditions as described before (Wagner, W., et al., Comparative characteristics of mesenchymal stem cells from human bone marrow, adipose tissue, and umbilical cord blood. Exp Hematol, 2005. 33(11): p. 1402-16.).

Briefly, bone marrow aspirates (10-30 ml) were collected in a syringe containing 10,000 IU heparin to prevent coagulation. The MNC fraction was isolated by density gradient centrifugation on Ficoll-Hypaque (d=1.077 g/cm³; Biochrom KG, Berlin, Germany) and seeded in tissue culture flasks at a density of 1x10⁶ cells/cm² (Nunc®-flasks with 75cm² by Nalge Nunc, Naperville, III., USA) for two days.

MSC were expanded in the commercially available Poietics human Mesenchymal Stem Cell Basal Medium (PT-3001, LONZA, Walkersville, MD, USA) following the manufacturer's instructions. 5,000 cells/cm² were plated in tissue flasks without any pre-coating. Culture medium was always changed twice per week. After reaching 80% confluence, MSC were trypsinized, counted with a Neubauer counting chamber (Brand, Wertheim, Germany), and re-seeded at 10⁴ cells/cm² for further expansion. If not indicated otherwise, sub-confluent MSC feeder layer (70-80%) of passage 4 to 6 was used in this study. Differentiation capacity into osteogenic, adipogenic and chondrogenic lineage of the MSC was confirmed.

For HSPC/MSC coculture experiments LTBMC medium was used, which is a basal HSPC culture medium described by Dexter et al.( Dexter, T.M., M.A. Moore, and A.P. Sheridan, Maintenance of hemopoietic stem cells and production of differentiated progeny in allogeneic and semiallogeneic bone marrow chimeras in vitro. J Exp Med, 1977. 145(6): p. 1612-6), consisting of 75% Iscove's modified Dulbecco's medium (IMDM; Invitrogen GmbH, Karlsruhe, Germany), 12.5% FCS, 12.5% horse serum (both Stemcell Technologies Inc., Vancouver, Canada), supplemented with 2 mM L glutamine, 100 U/ml penicillin/streptomycin (Invitrogen GmbH, Karlsruhe, Germany), and 0.05% hydrocortisone 100 (Sigma-Aldrich Chemie GmbH, Munich, Germany).

### RT and Real-Time RT-PCR

RNA concentration was determined using a lab on a chip RNA6000 Nano Series II kit (Agilent Technologies, Waldbronn, Germany) that was run on an Agilent 2100 Bioanalyzer instrument (Agilent Technologies, Waldbronn, Germany). The samples were diluted to a concentration of 0.4 µg/µl RNA, and 2.5 µg of total RNA from the samples was used for the reverse transcription. The reaction was performed using the Transcriptor High Fidelity cDNA synthesis kit (05081963001, Roche Diagnostics Deutschland GmbH, Mannheim, Germany) and according to the manufacturer's instructions. Briefly, in 50 µl containing 10 µl 5 x RT reaction buffer, 0.2 mM of each deoxynucleotide triphosphate, 20U recombinant RNase inhibitor, 5 mM dithiothreitol, 2.4 U High-Fidelity reverse transcriptase and 2.5 µM anchored oligo-d(T)₁₆ was used. The mixes were incubated for 30 min. at 45 °C, followed by 5 min at 85 °C and quick chilled on ice. Real-time PCR reactions were performed using the universal probe library (UPL) technique (Roche Applied Science, Mannheim, Germany).

The UPL technique makes use of 8-9mer probes that are labeled with FAM at the 5'-end and with a dark quencher at the 3'-end, and comprise 165 probes in total that cover the whole transcriptome of interest. Because of the shortness, each probe recognizes sequences that occur frequently in the transcriptome. But only one transcript is detected at a time because the ProbeFinder software (Roche Applied Science, Roche Diagnostics, Mannheim, Germany) designs a unique set of primers for the gene of interest. After the binding of the primers, the probes are hydrolyzed and fluorescence is generated which can be detected by the real-time instrument.

The reactions were performed on a Rotor-Gene 3000 instrument (Corbett Research, Sydney, Australia) in 25 µl containing 0.25 µl of a 10 µM solution of the specific probe (Roche Applied Science, Mannheim, Germany), 1.6 µl of a 10 mM dNTP solution (Amersham Biosciences, Piscataway, USA), 4 µl of a 12 µM UPL-primer solution, 12.5 µl of a 2 x QuantiFast Probe PCR kit buffer (Qiagen GmbH, Hilden, Germany) and 1 µl cDNA. The reactions were amplified for 40 cycles under the following conditions: 95°C for 30 s, 60°C for 30 s and 72°C for 30 s. We analyzed the expression of the genes listed in Table 1 in a semi-quantitative fashion using GAPDH as an internal standard.

**Table 1:**

| Primer | Forward | Reverse | UPL-probe |
|---|---|---|---|
| GAPDH | 5'- ctgacttcaacagcgacacc - 3' | 5'- tgctgtagccaaattcgttgt - 3' | #25 |
| CD7 | 5'- ggcggtgatctccttcct - 3' | 5'- aattcttatcccgccacga - 3' | #23 |
| CD14 | 5'- gttcggaagacttatcgaccat - 3' | 5'- acaaggttctggcgtggt - 3' | #74 |
| CD15 | 5'- gcgtgttggactacgagga - 3' | 5'- cgactcgaagttcatccaaac - 3' | #19 |
| CD33 | 5'- agtgaagacccacaggagga - 3' | 5'- ggccatgtaacttggacttctt - 3' | #71 |
| CD34 | 5'- tggagcaaaataagacctccag - 3' | 5'- aaggagcagggagcatacc - 3' | #52 |
| CD38 | 5'- cagaccgtaccttgcaacaa - 3' | 5'- aggtcatcagcaaggtagcc - 3' | #65 |
| CD44 | 5'- tcttcaacccaatctcacacc - 3' | 5'- gctgaagcgttatactatgactgg - 3' | #4 |
| CD90 | 5'- aggacgagggcacctacac - 3' | 5'- gccctcacacttgaccagtt - 3' | #22 |
| CD105 | 5'- ccactgcacttggcctaca - 3' | 5'- gcccactcaaggatctgg - 3' | #60 |
| CD166 | 5'- ggaggaatatggaatccaagg - 3' | 5'- ctgaatttacagtataccatccaagg -3' | #29 |
| c-kit | 5'- cggctctgtctgcattgtt - 3' | 5'- aacaggcacagctttgaagg - 3' | #79 |
| Nestin | 5'- cgttggaacagaggttgga - 3' | 5'- tgtaggccctgtttctcctg - 3' | #51 |
| p21 | 5'- cgaagtcagttccttgtggag - 3' | 5'- catgggttctgacggacat - 3' | #82 |
| SDF-1 | 5'- ccaaactgtgcccttcagat - 3' | 5'- tggctgttgtgcttacttgttt - 3' | #80 |
| Angpt-1 | 5'- gctaccatgctggagatagga - 3' | 5'- tctcaagtcgagaagtttgatttagt - 3' | #75 |
| IL-7 | 5'- tatgggcggtgagagctt - 3' | 5'- aggggaggaagtccaaagata - 3' | #15 |
| VCAM | 5'- tggaaaaaggaatccaggtg - 3' | 5'- aactgaacacttgactgtgatcg - 3' | #75 |
| SPP-1 | 5'- gagggcttggttgtcagc - 3' | 5'- caattctcatggtagtgagttttcc - 3' | #18 |
| CXCR-4 | 5'- cctgcctggtattgtcatcc - 3' | 5'- aggatgactgtggtcttgagg - 3' | #49 |

### Colony-Forming-Cell-Assay

For the colony forming cell-assay, 300 µl cell solution containing 2 * 10⁴ cells (HSPC or HSPC + MSC) were mixed with 3 ml methylcellulose (R&D Systems GmbH, Wiesbaden, Germany) and vortexed. For each HSPC batch 2.5 ml were pipetted in a 35 mm petri dish in duplicate. Samples were analyzed for the forming of colonies or for RT-PCR experiments at day 0, 7, 14 and 21 days prior to or after bioreactor culture, respectively.

Cells were re-seeded on a 24-well plate in serial dilution from 1:100 to 1:106 in 200 mL LTBMC medium. In each well, 500 mL methylcellulose (HSC-CFU lite with Epo, Miltenyi Biotec) was added. Final analyses were performed on day 14 and 21, respectively, depending on the end-point assay.

### Western Blot

The cells were washed with PBS and the supernatant aspirated. Afterwards, the cells were lysed by addition of 50 µl cell disruption buffer (Paris kit, Life Technologies, Darmstadt, Germany). Lysates were kept at -80°C until use. A maximum of 25 µl sample and 25 µl Lämmli buffer was then heated to 95°C for 5 minutes, cooled on ice and centrifuged with 14000g for 5 minutes. 20 - 40 µg total protein per sample was loaded onto an SDS-PAGE gel (10% pre-cast gel, NuSep, peqlab, Erlangen, Germany) and run with 50 mA and 120 V. Electrotransfer to PVDF membranes (Millipore, Schwabach, Germany) was accomplished using 300 mA and 70 V over night at 4°C in transfer buffer (25 mM Tris, 192 mM glycine, 10% MeOH).

PVDF-membranes were activated by wetting with methanol and washed with H₂0 before transfer. After transfer the membrane was incubated with blocking buffer (TBS + 0.2 % Tween-20 (TBST) + 10 % w/v non fat dry milk) for 60 min. at room temperature. Next, the membranes were incubated with the primary antibody against CD34 (1:50000 dilution, Epitomics 2749-1, Burlingname, CA, USA), CD38 (Abcam, Ab2577, Camridge, UK), and CD133 and incubated for 2h at room temperature. After washing (5 x TBST) for 10 min. the blots were incubated with the secondary antibody (goat-anti-mouse-peroxidase, A9917, Sigma-Aldrich, St. Louis, MO, USA) in a 1:80000 dilution in TBST + 10% non-fat dry milk for 1 h. The membranes were then incubated with a mixture of solution A and B of an enhanced chemiluminescence western blotting detection kit (GE-Healthcare, RPN2132, Freiburg, Germany. Finally, the blots were exposed to x-ray films for 30s to 2 min and developed (Cawomat 2000 IR, Cawo Photochemische Fabrik GmbH, Schrobenhausen, Germany).

### Widefield and confocal laser scanning microscopy imaging

KITChips were seeded with a cell population mix of MSC and HSPC (typically 300,000 MSC and 200,000 HSPC per KITChip) after the chips have been coated with collagen I (from rat tail, SIGMA, C-7661) over night. 1-3 days after seeding, the cells on the KITChip were carefully washed once with PBS and fixed subsequently with 4% PFA for 15min at room temperature. Depending on the staining for surface markers or intracellular proteins a permeabilization step with 0.2% Triton X-100 was performed. Before IF staining, the cell container area was cut out from the whole KITChip with a surgical scalpel for ease of further processing.

The KITChip was incubated with 100-200µl of the primary antibody solution for 1 h, then washed three times for 5 min each in PBS. Secondary fluorescently labelled antibody staining was done by incubation with 100-200ml for 1 h. After further three washing steps nuclear staining was performed with 500µl Hoechst (Hoechst 33342, 1µM) for 5 min followed by again final three times washing for 5 min each in PBS. Samoles were stored in PBS at 4°C until microscopic image acquisition.

Images were acquired either with an Olympus IX70 microscope equipped with a Colorview III camera under the control of the AnalySIS software from SIS (Soft Imaging System, Münster, Germany) or a Keycence BIOREVO BZ-9000 microscope. Object lenses with 20x, 40x and 60x magnification were used.

Additionally, confocal images were acquired at the Nikon Imaging Center at Heidelberg University with a Nikon C1Si-CLEM spectral imaging confocal laser scanning system using a 60x objective. For 3D image reconstruction of cells, z-stacks with 40 slices were acquired. Further processing and analysis of images and composition of image figures was done with ImageJ and Photoshop, respectively.

### Statistics

All experiments were performed in triplicates or greater unless otherwise indicated. For statistical comparison the Mann-Whitney U-test was performed. Results with p-values of <0.05 were considered as being statistically different from controls.

## Claims

1. A three-dimensional (3D) model of a hematopoietic stem cell and progenitor cells (HSPC) niche comprising:
(a) a microcavity array having at least one microcavity
(b) a 3D mesh formed by HSPC-supporting stroma cells in the at least one microcavity.

2. The 3D model of a HSPC niche of claim 1, wherein the HSPC-supporting stroma cells are mesenchymal stromal cells (MSC).

3. The 3D model of a HSPC niche of claim 1 or 2, wherein the microcavity has a volume of 10 to 100 nl.

4. The 3D model of a HSPC niche of any of the above claims, wherein the microcavity has a size of 200 to 400 µm x 200 to 400 µm x 200 to 400 µm (height x depth x width).

5. The 3D model of a HSPC niche of any of the above claims, further comprising HSPC.

6. A method of cultivating hematopoietic stem cells (HSPC) comprising the step of cultivating HSPC in the three-dimensional (3D) model of a HSPC niche as defined in any of the above claims.

7. The method of claim 6, comprising the steps of:
(a) providing a microcavity array having at least one microcavity,
(b) seeding a mixture of stroma cells and HSPC at the same time into the at least one microcavity,
(c) cultivating the cells of step (b).

8. The method of claim 6, comprising the steps of:
(a) providing a microcavity array having at least one microcavity having a porous bottom,
(b) seeding HSPC-supporting stroma cells in the at least one microcavity,
(c) cultivating the HSPC-supporting stroma cells of step (b) until a 3D mesh is formed,
(d) seeding HSPC into the 3D mesh formed by the HPC-supporting stroma cells in step (c), and
(e) cultivating the HSPC of step (d).

9. The method of any of claims 6 to 8, wherein the HSPC-supporting stroma cells are mesenchymal stromal cells (MSC).

10. The method of any of claims 6 to 9, wherein the cultivation of cells is carried out in a closed circulation system comprising the microcavity array in a bioreactor, a cassette pump, a gas mixing station, and a medium reservoir.

11. The method of any of claims 6 to 10, wherein the cultivation of cells is carried out using superfusion of the cells with medium, wherein the medium flows in parallel to the assay surface, and/or using perfusion of the cells with medium, wherein the medium enters the chip through the porous bottom of the at least one microcavity.

12. The method of any of claims 6 to 11, wherein the MSC and the HSPC are derived from the same species.

13. The method of claim 12, wherein the MSC and the HSPC are of human origin.

14. The method of any of claims 6 to 13, wherein the cells are cultivated in a microbioreactor system that is actively perfused and/or superfused with medium.

15. Use of a three-dimensional (3D) model of a hematopoietic stem cell (HSPC) niche as defined in any of claims 1 to 5 for cultivating HSPC.


SEQUENCE LISTING

<110>  Karlsruher Institut für Technologie
       Universitätsklinikum Heidelberg

<120>  In vitro co-culture system

<130>  K 4642

<160>  40

<170>  PatentIn version 3.5

<210>  1
<211>  20
<212>  DNA
<213>  artificial sequence

<220>
<223>  primer

<400>  1
ctgacttcaa cagcgacacc                                                   20


<210>  2
<211>  21
<212>  DNA
<213>  artificial sequence

<220>
<223>  primer

<400>  2
tgctgtagcc aaattcgttg t                                                 21


<210>  3
<211>  18
<212>  DNA
<213>  artificial sequence

<220>
<223>  primer

<400>  3
ggcggtgatc tccttcct                                                     18


<210>  4
<211>  19
<212>  DNA
<213>  artificial sequence

<220>
<223>  primer

<400>  4
aattcttatc ccgccacga                                                    19


<210>  5
<211>  22

<212>  DNA
<213>  artificial sequence

<220>
<223>  primer

<400>  5
gttcggaaga cttatcgacc at                                                22


<210>  6
<211>  18
<212>  DNA
<213>  artificial sequence

<220>
<223>  primer

<400>  6
acaaggttct ggcgtggt                                                     18


<210>  7
<211>  19
<212>  DNA
<213>  artificial sequence

<220>
<223>  primer

<400>  7
gcgtgttgga ctacgagga                                                    19


<210>  8
<211>  21
<212>  DNA
<213>  artificial sequence

<220>
<223>  primer

<400>  8
cgactcgaag ttcatccaaa c                                                 21


<210>  9
<211>  20
<212>  DNA
<213>  artificial sequence

<220>
<223>  primer

<400>  9
agtgaagacc cacaggagga                                                   20


<210>  10
<211>  22
<212>  DNA
<213>  artificial sequence

<220>
<223>  primer

<400>  10
ggccatgtaa cttggacttc tt                                                22


<210>  11
<211>  22
<212>  DNA
<213>  artificial sequence

<220>
<223>  primer

<400>  11
tggagcaaaa taagacctcc ag                                                22


<210>  12
<211>  19
<212>  DNA
<213>  artificial sequence

<220>
<223>  primer

<400>  12
aaggagcagg gagcatacc                                                    19


<210>  13
<211>  20
<212>  DNA
<213>  artificial sequence

<220>
<223>  primer

<400>  13
cagaccgtac cttgcaacaa                                                   20


<210>  14
<211>  20
<212>  DNA
<213>  artificial sequence

<220>
<223>  primer

<400>  14
aggtcatcag caaggtagcc                                                   20


<210>  15
<211>  21
<212>  DNA
<213>  artificial sequence

<220>
<223>  primer

<400>  15
tcttcaaccc aatctcacac c                                                21

<210>  16
<211>  24
<212>  DNA
<213>  artificial sequence

<220>
<223>  primer

<400>  16
gctgaagcgt tatactatga ctgg                                             24

<210>  17
<211>  19
<212>  DNA
<213>  artificial sequence

<220>
<223>  primer

<400>  17
aggacgaggg cacctacac                                                   19

<210>  18
<211>  20
<212>  DNA
<213>  artificial sequence

<220>
<223>  primer

<400>  18
gccctcacac ttgaccagtt                                                  20

<210>  19
<211>  19
<212>  DNA
<213>  artificial sequence

<220>
<223>  primer

<400>  19
ccactgcact tggcctaca                                                   19

<210>  20
<211>  18
<212>  DNA
<213>  artificial sequence

<220>
<223>  primer

<400>  20
gcccactcaa ggatctgg                                                    18

<210>  21
<211>  21
<212>  DNA
<213>  artificial sequence

<220>
<223>  primer

<400>  21
ggaggaatat ggaatccaag g                                                 21


<210>  22
<211>  26
<212>  DNA
<213>  artificial sequence

<220>
<223>  primer

<400>  22
ctgaatttac agtataccat ccaagg                                            26


<210>  23
<211>  19
<212>  DNA
<213>  artificial sequence

<220>
<223>  primer

<400>  23
cggctctgtc tgcattgtt                                                    19


<210>  24
<211>  20
<212>  DNA
<213>  artificial sequence

<220>
<223>  primer

<400>  24
aacaggcaca gctttgaagg                                                   20


<210>  25
<211>  19
<212>  DNA
<213>  artificial sequence

<220>
<223>  primer

<400>  25
cgttggaaca gaggttgga                                                    19


<210>  26
<211>  20

<212>  DNA
<213>  artificial sequence

<220>
<223>  primer

<400>  26
tgtaggccct gtttctcctg                                                    20


<210>  27
<211>  21
<212>  DNA
<213>  artificial sequence

<220>
<223>  primer

<400>  27
cgaagtcagt tccttgtgga g                                                  21


<210>  28
<211>  19
<212>  DNA
<213>  artificial sequence

<220>
<223>  primer

<400>  28
catgggttct gacggacat                                                     19


<210>  29
<211>  20
<212>  DNA
<213>  artificial sequence

<220>
<223>  primer

<400>  29
ccaaactgtg cccttcagat                                                    20


<210>  30
<211>  22
<212>  DNA
<213>  artificial sequence

<220>
<223>  primer

<400>  30
tggctgttgt gcttacttgt tt                                                 22


<210>  31
<211>  21
<212>  DNA
<213>  artificial sequence

<220>
<223>  primer

<400>  31
gctaccatgc tggagatagg a                                                21


<210>  32
<211>  26
<212>  DNA
<213>  artificial sequence

<220>
<223>  primer

<400>  32
tctcaagtcg agaagtttga tttagt                                           26


<210>  33
<211>  18
<212>  DNA
<213>  artificial sequence

<220>
<223>  primer

<400>  33
tatgggcggt gagagctt                                                    18


<210>  34
<211>  21
<212>  DNA
<213>  artificial sequence

<220>
<223>  primer

<400>  34
aggggaggaa gtccaaagat a                                                21


<210>  35
<211>  20
<212>  DNA
<213>  artificial sequence

<220>
<223>  primer

<400>  35
tggaaaaagg aatccaggtg                                                  20


<210>  36
<211>  23
<212>  DNA
<213>  artificial sequence

<220>
<223>  primer

<400>  36
aactgaacac ttgactgtga tcg                                               23


<210>  37
<211>  18
<212>  DNA
<213>  artificial sequence

<220>
<223>  primer

<400>  37
gagggcttgg ttgtcagc                                                     18


<210>  38
<211>  25
<212>  DNA
<213>  artificial sequence

<220>
<223>  primer

<400>  38
caattctcat ggtagtgagt tttcc                                             25


<210>  39
<211>  20
<212>  DNA
<213>  artificial sequence

<220>
<223>  primer

<400>  39
cctgcctggt attgtcatcc                                                   20


<210>  40
<211>  21
<212>  DNA
<213>  artificial sequence

<220>
<223>  primer

<400>  40
aggatgactg tggtcttgag g                                                 21